# EUROPEAN PATENT APPLICATION

(11) **EP 4 813 287 A1**
(43) Date of publication of application: **30.09.2026**
(21) Application number: 25165750.8
(22) Date of filing: 24.03.2025
(51) Int. Cl.: A61B 5/02, A61B 5/11, A61B 5/16, A61B 5/00

(54) **METHOD FOR DETECTING A SENSORY OVERLOAD OF A USER WITH A HEARING DEVICE**

(71) Applicant: Sonova AG, 8712 Stäfa (CH)
(72) Inventor: STUMPF, Nina, 8712 Stäfa (CH); MARNAT, Marguerite, 8712 Stäfa (CH); WENHART, Teresa, 8712 Stäfa (CH); KNOBEL, Samuel Elia Johannes, 8712 Stäfa (CH); HERBERT, Nicholas, 8712 Stäfa (CH); MURTOLA, Evengy, 8712 Stäfa (CH)

(57) **Abstract**

Method for detecting a sensory overload of a user with a hearing device comprising the following steps of, measuring an acoustic property value of an audio input to detect whether there is acoustically induced stress, and measuring a motion property value to detect whether there is behavioral stress of the user, and selecting an acoustic stimulus that is configured to modify a sound processing of the audio input and convert it to an audio output if the acoustic property value is equal to or above a predetermined acoustic property threshold value and/or if the motion property value is equal to or above a predetermined motion property threshold value, and modifying the sound processing with the acoustic stimulus, and re-measuring the acoustic property value and the motion property value and verifying whether the re-measured motion property value is lower than the motion property value measured at step b).

## Description

### TECHNICAL FIELD

The disclosure relates to a method for detecting a sensory overload of an end user with a hearing device and to mitigate the negative effect of stress for the end user.

### BACKGROUND

Today's hearing devices are sophisticated devices that are capable of way more functionalities than merely amplifying an incoming sound signal. Many hearing devices have sensors built in such as accelerators to interpret tap commands and the like. Moreover, several attempts have been made to fit hearing devices with further sensors to gather other data such as health-related data, for example.

Hearing devices may be used to improve the hearing capability or communication capability of a user, for instance by compensating a hearing loss of a hearing-impaired user, in which case the hearing device is commonly referred to as a hearing instrument such as a hearing aid or hearing prosthesis. A hearing device may also be used to output sound based on an audio signal which may be communicated by a wire or wirelessly to the hearing device. A hearing device may also be used to reproduce a sound in a user's ear canal detected by an input transducer such as a microphone or a microphone array. The reproduced sound may be amplified to account for a hearing loss, such as in a hearing instrument, or may be output without accounting for a hearing loss, for instance to provide for a faithful reproduction of detected ambient sound and/or to add audio features of an augmented reality in the reproduced ambient sound, such as in a hearable. A hearing device may also provide for a situational enhancement of an acoustic scene, e.g. beamforming and/or active noise cancelling (ANC), with or without amplification of the reproduced sound. A hearing device may also be implemented as a hearing protection device, such as an earplug, configured to protect the user's hearing. Different types of hearing devices configured to be worn at an ear include earbuds, earphones, hearables, and hearing instruments such as receiver-in-the-canal (RIC) hearing aids, behind-the-ear (BTE) hearing aids, in-the-ear (ITE) hearing aids, invisible-in-the-canal (IIC) hearing aids, completely-in-the-canal (CIC) hearing aids, cochlear implant systems configured to provide electrical stimulation representative of audio content to a user, a bimodal hearing system configured to provide both amplification and electrical stimulation representative of audio content to a user, or any other suitable hearing prostheses. A hearing system comprising two hearing devices configured to be worn at different ears of the user is referred to as a binaural hearing device. A hearing system may also comprise a hearing device, e.g., a single monaural hearing device or a binaural hearing device, and a user device, e.g., a smartphone and/or a smartwatch, communicatively coupled to the hearing device.

Hearing devices are often embedded in a hearing system in conjunction with communication devices, such as smartphones or tablets, for instance when listening to sound data processed by the communication device and/or during a phone conversation operated by the communication device. More recently, communication devices have been integrated with hearing devices such that the hearing devices at least partially comprise the functionality of those communication devices. A hearing system may comprise, for instance, a hearing device and a communication device.

Advanced audio signal processing techniques have been developed to improve a hearing experience for users in various fields. For example, hearing instruments rely on audio signal processing to enhance auditory perception for individuals with hearing impairments.

Several concepts are promoted to mitigate the stress perception of a human. In the field of hearing devices, there are several publications promoting the use of electroencephalography (EEG) for monitoring a mental load and the fatigue of an end user. US2021/0235203A1 is a representative of such prior art. A major problem of such a scheme resides in that EEG requires sensor electrodes that are inconvenient or impractical to wear in daily life, even if they are built-into a pair of glasses or the like.

Another attempt is made by US2023/0049262A1 that promotes an IoT-connected wearable device such as a pair of glasses that is fitted with eye-tracking sensors and connected with a datastore where sensual or physiological sensory thresholds are stored. When in use, the system verifies whether the sensory input is exceeding a user-specific threshold, and an intervention is configured to provide the user relief from distractibility, inattention, anxiety, fatigue, or sensory issues. That intervention is then provided to the user of that wearable device. The intervention comprises a filtering, in real-time, of an audio signal presented to the user or an optical signal presented to the user. The problem with such a wearable device resides again in the sensors required and the moderate wearing comfort, just to name a few.

One might think that using the sound classifier information might suffice to address and mitigate the stress reduction. Many hearing devices use so-called classifiers. The classifier analyzes sound scenes based on the audio input arriving at the hearing device and passes a sound classifier information to the system steering of the hearing device. The system steering of the hearing device then decides on what to do with the sound classifier and how to process the audio input signal. Exemplary sound scenes are "streaming" for listening to music or television, "party mode" for places where many people gather and there is a plurality of simultaneous talking, "restaurant" for a situation where there are not only voices of several people but also sounds of clinking glasses, cutlery and a rather high background noise.

Unfortunately, all end users are individuals whose stress tolerance levels and stress perceptions are different. For that reason, it is not reasonable to always reduce the gain of a stressful sound when such an acoustic condition arises.

For all these reasons, there is an ongoing need for a method that is executable in a hearing device, which method can detect stress when it exceeds a threshold and mitigate the stress only when there is a need. The method shall also be able to detect the primary stress type and to fight it as efficiently as possible.

### SUMMARY

Since today's hearing devices have more and more sensors built-in, it is an aim of the present disclosure to make further use of these sensors and to provide a solution to detect a sensory overload of an end user that is wearing a hearing device anyway, and a possibility to mitigate the negative effect of stress for that end user.

Sensory overload results in a stress response which can be measured by suitable sensors. The difficulty resides in distinguishing the presence of a sensory overload from other causes, to detect whether the sensory overload is triggered by the acoustical sound scene the end user is exposed to and that can cause acoustically induced stress to the end user, or whether the sensory overload is predominantly a result of emotional stress of the end user. Emotional stress is a relevant factor contributing to a sensory overload and sensory overload causes emotional stress in turn. A further difficulty resides in that acoustically induced stress can trigger emotional stress for the end user such that there is eventually a mix of stress types to be fought. Measuring the acoustic property value and comparing it with an acoustic property threshold value contributes positively to the robustness of the method.

Emotional stress is a response of emotional or physical tension caused by a physiological response in the human body. It can be triggered by situations of danger, threat, and loss of personal security or by internal conflicts, frustrations, loss of self-esteem, grief or a difficult talk are just a few examples. Emotional stress can produce feelings of psychological strain and uneasiness. A general restlessness usually goes in hand with higher motion levels than usual and are thus a good indicator for the presence of an emotional stress overload. Increased head movement activity or a change in the head movement pattern and speed are classic indicators of the presence of emotional stress.

Acoustically induced stress, also referred to as environmental stress having an acoustic component is a response of physical tension caused by a physiological response in the human body. It can be triggered by regular loud situations like a sledgehammer or a chainsaw, but also irregular loud sounds like bursts of a gun, a power drill or the like. Not only loud, but also comparatively silent sound like a shrieking mechanism of a barn door hinge or a scratching sound may be perceived as annoying and therefore stressful by a human. Besides the loudness or high noise levels, there are further factors like the sound pattern, the speech intensity in case of a spoken message, a sound pitch, and a sound-to-noise ratio (SNR) that is below a certain threshold, or sound that is arriving from different directions relative to the end user and leading to a complex sound scene can lead to stress for a human.

It was found that an acoustic stimulus that is mixed to the processed sound signal that forms the output of the hearing device can positively contribute to mitigating both emotional stress as well as acoustically induced stress. It was further found that depending on the end user preferences, there may be different stimuli for mitigating emotional stress as well as acoustically induced stress. Moreover, there may be more than one stimulus that is suitable to reduce the stress level of emotional stress and acoustically induced stress, each.

The above-mentioned improvement of the stress detection and mitigation is achievable by a method for detecting a sensory overload of a user with a hearing device by means of a detection system that comprises the following steps:
a) Measuring an acoustic property value of an audio input via the hearing device, which acoustic property is serving as an identifier of the presence of an acoustically induced stress,
b) Measuring a motion property value serving as an identifier of the presence of a behavioral stress of the user,
c) Selecting an acoustic stimulus that is configured to modify a sound processing of the audio input and convert it to an audio output if the acoustic property value is equal to or above a predetermined acoustic property threshold value and/or if the motion property value is equal to or above a predetermined motion property threshold value,
d) Modifying the sound processing of the audio input by applying the acoustic stimulus,
e) Re-measuring the acoustic property value and the motion property value and verifying whether the re-measured motion property value is lower than the motion property value measured at step b).

The method is performed by an algorithm that can be activated or deactivated in the hearing device. The acoustic property can be the sound pressure level (loudness) of a sound. Other acoustic properties are the sound pattern, a speech intensity or a sound pitch. More sophisticated methods do not only take the loudness into account but also consider at least one of a sound pattern and a sound pitch. These acoustic properties are available during the digital sound processing anyway. They only need to be gathered for the present, additional purpose.

The motion property value is measured by an accelerometer or a gyroscope, for example. Alternative of complementary motion property sensors are electrooculography (EOG) that tracks eye movements. Such motions sensors can be integrated in wearables or part of wearables such as a wrist band, a smartwatch, a smartphone attached snugly to the body of the end user in places that express emotional stress. An accelerometer built-into a hearing device is a preferred choice as it can also be used to detect a tap command.

The verification on whether the acoustic property value measured by the acoustic sensor is above, at or below a predetermined acoustic property threshold value is performed by an intelligence unit located in the hearing device, too. The predetermined acoustic property threshold value is stored in a lookup table provided in the hearing device, too. The lookup table is part of a mathematical stress model. The predetermined acoustic property threshold value is not a value that is defined by a health care professional (HCP) during an initial fitting process and remains there unchanged. The method is able to learn end user preferences and is able to adapt to changed user preferences over time one the method is operated in an interactive learning mode or a self-learning mode. To allow the end user to profit from an effective stress mitigation opportunity according to the present method, it is advantageous if the HCP defines during a fitting session with the end customer at least one acoustic stimulus that shall be defined to address acoustically induced stress when it arises. It is recommended, albeit not indispensable to store more than one acoustic stimulus in the look up table that is directed to acoustically induced stress. It is recommended to select at least two acoustic stimuli that are different to one another with respect to their audio information. This because there may be acoustic situations where an acoustic stimulus may not derive the desired soothing effect such that the method can deactivate the first acoustic stimulus and activate a second, alternative or spare acoustic stimulus and try mitigating the acoustically induced stress that way.

In a preferred embodiment, the HCP defines together with the end user the acoustic property threshold value and stores it via the fitting software in the look up table of the hearing device of the end user. It is possible that the end user provides a sound record to the HCP that creates the acoustic stimulus based on that sound record, next. Alternatively, it may be convenient for the HCP as well as for the end user alike, if the fitting software has a predefined selection of acoustic stimuli available to be implemented in the hearing device. Alternatively, the predefined selection of acoustic stimuli available is already contained in the look up table in the hearing device and the HCP just selects and activates them based on interviewing the end user and/or asking her/him on whether some predefined sound types forming the acoustic stimulus are preferable compared to others. Exemplary acoustic stimuli besides music are surf of the sea, birds, rustling of leaves, wind passing.

As to the acoustic stimuli, recent research revealed that similar music can be used to stay focused when concentration is needed and to relax, e.g. with the purpose to fall asleep or reduce excitation of the nervous system, the latter of which is a precondition for both relaxation and falling asleep.

Albeit pleasantness of music is very individual, numerous research has shown that slow, calm, silent music is on average considered as relaxing compared to fast, loud music with energetic rhythms. Energizing music leads to a slight stress response (e.g. reduction of heart rate variability). Corresponding to the increase in the heart rate, listening to exciting music (compared with tranquilizing music) is associated with a reduction of the heart rate variability, including reductions of both low-frequency and high-frequency power of the HRV.

Recent findings also suggest effects of music-evoked emotions on regional activity of the heart, as reflected in electrocardiogram amplitude patterns. In patients with heart disease (like other patient groups), music can reduce pain and anxiety, associated with lower heart rate and lower blood pressure. In general, the effects of music on the heart are small, and there is great inhomogeneity among studies regarding methods, findings, and quality. Further papers indicate the relaxing effect of relaxing music on patients.

To avoid the risk that the end user is falling asleep because the acoustic stimulus is overly relaxing, calming music in a stimulus should not be so calming, or relaxing that it has a hypnotic effect.

Further, the duration of the time length the stimulus shall be applied can be personalized. In exemplary embodiments, the acoustic stimulus is played for some minutes only. In a further embodiment, the application of the acoustic stimulus can be uninterrupted or intermediately with some breaks.

The verification on whether the motion property value measured by the acoustic sensor is above, equal to, or below a predetermined motion property threshold value is performed by an intelligence unit located in the hearing device. Depending on the architecture of the hearing device, it may be the same intelligence unit that verifies whether the acoustic property value measured by the acoustic sensor is above, at or below a predetermined acoustic property threshold value.

The predetermined motion property threshold value is stored in a lookup table provided in the hearing device. The lookup table is part of a stress model, too. The predetermined motion property threshold value is not a value that is defined by a health care professional (HCP) during an initial fitting process and remains there unchanged. The method is configured such that it is able to learn end user preferences and is able to adapt to changed user preferences over time one the method is operated in an interactive learning mode or a self-learning mode. To allow the end user to profit from an effective stress mitigation opportunity according to the present method, it is advantageous if the HCP defines during a fitting session with the end customer at least one acoustic stimulus that shall be defined to address emotional stress when it arises. It is recommended, albeit not indispensable to store more than one acoustic stimulus in the look up table that is directed to emotional stress. Again, it is recommended to select at least two acoustic stimuli that are different to one another with respect to their audio information. This because there may be emotional stress situations where a single acoustic stimulus may not derive the desired soothing effect such that the method can deactivate the first acoustic stimulus and activate a second, alternative or spare acoustic stimulus and try mitigating the emotional stress that way.

If the accuracy of detecting the presence of emotional stress shall be increased, then it is advisable to measure more than one motion property value, for example the motion intensity as well as the pattern of head movements.

It is recommended that the end user then trains the mathematical stress model such that it can meet her/his preferences and requirements even more accurately and situation-dependent than with the initial settings in the stress model installed by the HCP.

In a preferred embodiment, the HCP defines together with the end user the motion property threshold value in a fitting session and stores it via the fitting software in the look up table of the hearing device of the end user, again.

The HCP can tune the default stress profile in the stress model based on parameters such as age or known medical condition during the fitting.

When using the hearing device for the first time after the fitting process is concluded, the method will apply the potentially appropriate acoustic stimulus in a specific stress situation once that it detects a stress response of the end user. It is possible to re-adjust or even replace not only the type but also the intensity of the stimuli any time after the initial fitting during a follow-up fitting session or even via an app running on a mobile device such as a mobile phone or a table that is connectable to the hearing device by the end user.

The modification of the audio input at step d) is a so-called reduction of the sound field, regardless of whether the stress response was predominantly an acoustically induced stress one, an emotional stress one or a mix between the two. In case that there is a mix of acoustically induced stress and emotional stress and where both the acoustic property value in the audio input is equal to or above the predetermined acoustic property threshold value and where the motion property value is equal to a above the predetermined motion property threshold value, then the acoustic stimulus is preferably a mix between a stimulus that is predefined to be applied in case of acoustically induced stress and a stimulus that is predefined to be applied in case of emotional stress. In an embodiment, the mix is achievable by a weighted adding of the stimuli. In case that the method cannot identify whether the end user perceives predominantly acoustically induced stress or emotional stress, then the stimulus that is dedicated to mitigating acoustically induced stress shall be applied in step d) of the above-mentioned method when modifying the sound processing.

If the intelligence unit detects that the acoustic property value in the audio input is below the predetermined acoustic property threshold value and that the motion property value is below a predetermined motion property threshold value, no stress mitigation is required, and the method re-starts at step a) again. Step c) is not consuming a lot of electric energy at all.

In a less favored embodiment of the present invention, the acoustic property value and the motion input measured in steps a) and b) are merged to a combined input value that is then compared with a combined threshold value. Such an embodiment is less favored because it allowed an even less accurate differentiation in the detection of acoustically induced and emotional stress, especially when the method is operated in the automatic mode where no learning is involved.

As mentioned above, emotional stress often accompanies acoustically induced stress. Hence, a mix of an acoustic stimulus directed to address acoustically induced stress, and an acoustic stimulus directed to address emotional stress forms the most effective option. In such a case, the acoustic stimulus comprises at least one of a first mask that is configured to mitigate acoustically induced stress, and a second mask that is configured to mitigate emotional stress. The first mask and the second mask can be the same and lead eventually to the same acoustic stimulus if the end user just selected a single preferred stimulus to mitigate both acoustically induced stress as well as emotional stress.

Since measuring the acoustic property value in the audio input and the motion property value only does not allow for an exact differentiation between acoustically induced stress and emotional stress, also a physiologic property value is better considered to increase the accuracy of the stress-type analysis outcome. Expressed in other words, the physiological property value measurement contributes substantially to the reliability of the detection of stress of the end user.

In such an embodiment, the method is characterized by an additional step of measuring a physiologic property value in addition to the motion property value in step b) as well, and by considering said physiologic property value when selecting the acoustic stimulus at step c), and by re-measuring the physiologic property value at step e) in addition to the re-measuring of the motion property value, as well as by verifying whether the re-measured physiologic property value is lower than the physiologic property value measured at step b) of the above-mentioned method.

Re-measuring the physiologic property value after applying the acoustic stimulus and comparing it with the measured physiologic property value before applying the acoustic stimulus to detect a decrease of the stress is conditional for operating the method in the self-learning mode. There is no situation believed to be existing where there is an acoustic property value that is below an acoustic property threshold value, the motion property value being below the acoustic property threshold value but where there is a physiologic stress response that was equal to above a physiologic property threshold value. Hence, it is not necessary to start the full process of the method and measure the physiologic property value if the acoustically induced property value and the motion property value are below their threshold values, each. Such a cascaded activation of sensors and intelligence is also recommendable in the light of a low power consumption of the method. A low power consumption is relevant since the electrical power available in a hearing device is limited by the capacity of the battery. That battery can be a re-chargeable battery of a conventional battery, such as a copper-zinc battery that needs to be replaced in case it is consumed.

The physiological property value is measured by a sensor for measuring at least one activity in the following technical fields: Photoplethysmography (PPG), electrocardiography (ECG), electrodermal activity (EDA), i.e., measuring the skin conductance, electroencephalography (EEG), heart rate variability (HRV), heart rate (HR) and the temperature (skin temperature), skin conductance. EEG, PPG and EDA sensors can be integrated in wearables like chest straps, smart watches, headbands or the like. However, it is also possible to integrate such sensors in the hearing device such that they are not only invisible to third parties but also convenient since there is no additional wearable device required.

HRV, HR and PPG values are comparatively easy and reliably obtainable whereas skin conductance and EEG values are often weaker and thus less clear, harder to identify and thus less reliable to interpret. Hence, HRV, HR and PPG sensors are a preferred choice to be integrated into a hearing device for implementing the method according to the present invention.

It proved advantageous if the method is not stopped immediately after the re-measured acoustic property value, motion property value and/or physiologic property value falls below the measured acoustic property value, motion property value and/or physiologic property value measured at step a) or b) of the method previously but if the stimulus is continued to be applied for a further while. This is particularly beneficial in case where the stress level in a specific situation is just slightly above the threshold, then declines as long as the acoustic stimulus is applied but rises above the threshold again as soon as the acoustic stimulus is not applied any longer.

Hence, the method is refined in that the acoustic stimulus is re-selected at step c) and re-applied at step d) of the above-mentioned method in a new cycle of the method that is following a previous cycle of the method if the re-measured acoustic property value is lower than the acoustic property value, the re-measured motion property value is lower than the motion property value and the physiologic property value measured at step a) and b) of the previous cycle of the method, each.

The re-application of the acoustic stimulus can be for a predefined number of repetitions (for example 50 times with a gap of 2 seconds between two applications) or an issuance over a predetermined amount of time (for example 3 minutes longer than the end of loud situation that led to the sensory overload).

Once the re-measured acoustic property value, motion property value, and if available the physiologic property value stays below their dedicated threshold for a predefined time, the process of the stress monitoring begins at step a) anew. Since stress situations typically do not change within a few seconds from one state to the other, it is advisable to delay the re-start of the process at step a) of the method for some time, for example by 30 seconds, or even by some minutes. Such a delay does not only contribute to power saving but avoids exposing the end user to a constant exposure of acoustic stimuli.

It is possible that the acoustic stimulus selected at step c) would be suitable for mitigating the stress for the end user immediately but it just too feeble to lead to a substantial desired stress mitigation instantly. One must consider that the body response to a stress mitigation takes its time until it becomes measurable and distinguishable properly. Hence, it is better to apply the same acoustic stimulus as applied in a prevailing cycle of the method but with an increased intensity, for example an increased loudness by 0.5 dB before replacing the stimulus by an alternative acoustic stimulus as the latter procedure might be considered as annoying or even stressful to the end user. Alternative ways to increase the intensity of the acoustic stimulus reside in altering the pitch, length, speed, sentiment of the content of the acoustic stimulus.

In such an embodiment, the acoustic stimulus is re-selected at step c) and re-applied with an increased intensity at step d) in a new cycle of the above-mentioned method if the re-measured acoustic property value, re-measured motion property value, and if available the re-measured physio-logic property value at step e) of the new cycle of the method is equal to or higher than the acoustic property value, the motion property value, and if available the physiologic property value measured at step a) and b) of a previous cycle of the method, respectively.

It is possible that an acoustic stimulus does not derive the desired stress mitigation effect to the end user despite the end user identified to the HCP at the time of the initial fitting or later that such an acoustic stimulus is preferred and has the potential to mitigate the stress perceived. In such cases, it is beneficial to have a second, alternative acoustic stimulus at hand that can replace the initial acoustic stimulus in case that initial acoustic stimulus did not work over a predefined number of applications or a predefined period.

In such a case, the method is further refined in that the acoustic stimulus is replaced at step c) of the above-mentioned method in a new cycle of the method by an alternative acoustic stimulus, provided that the re-measured acoustic property value of the new cycle of the method is equal to or higher than the previous acoustic property value, the re-measured motion property value of the new cycle of the method is equal to or higher than the previous motion property value, and - where available - the re-measured physiologic property value of the new cycle of the method is equal to or higher than the previous physiologic property value measured, respectively. That refinement shall only take place if several repetition cycles with the acoustic stimulus exceeded a predefined repetition threshold.

In an exemplary summary, a first acoustic stimulus that is based on a breaking of waves sound is replaced by a second stimulus that is based on soft classic piano music from Mozart.

As mentioned above, there may be more than one alternative acoustic stimulus. The number of alternative stimuli in the stress model depends on the number of stimuli defined during the fitting process by the HCP and/or that was uploaded by the end user in a post-fitting process via a user interface, for example an app on a smartphone, for example, to the stress model. Alternative, although less favored communication means for allowing an end user feedback to the stress model are formed by a tap control scheme, a voice control scheme, or via an interface located at the HCP's premise only.

The innovative method may further be refined by an additional step of aborting the re-execution of the method if a predefined further repetition threshold is exceeded.

Such an abortion of the process where neither the acoustic stimulus nor the alternative acoustic stimulus led to the intended decrease of the stress level of the end user in an automatic mode of the method may be suitable if the stress detection was defective. When the method is run in the self-learning mode or the interactive learning mode that will be explained later in more detail, the stress model will not be updated for that particular stress situation (both with respect to the acoustic sound scene, the motion state and the physiologic values, if available, in order to avoid an amendment of the stress model with data that is potentially faulty.

When reverting to the automatic mode in which the present method can be run, the steps of the method are repeated until an operating mode other than the automatic mode is selected, the hearing device is shut off, and/or in that the method re-starts at step a) in case the re-measured physiologic property value at step e) of an initial cycle of the method is lower than a predefined physiologic property threshold value. As mentioned before, the method cycle may restart immediately or after a delay.

There is no leaming/updating of the mathematical stress model in the automatic mode. In the automatic mode, the lessons leamed during a previous learning phase, regardless of whether by self-learning or interactive learning or even just based on the initial settings of the HCP are applied in those situations that were learned or defined to be stressful and that are now stored in the stress model.

As mentioned previously, the present method is further operable in a self-learning mode, where a stress model is updated with the following information after executing step e) the method:
- The acoustic property value measured at step a);
- The motion property value measured at step b);
- The physiologic property value measured at step b);
- what acoustic stimulus was applied in what intensity in step d);
- The re-measured acoustic property value measured at step e);
- The re-measured motion property value measured at step e);
- The re-measured physiologic property value measured at step e);
- A conclusion derived based on a result of the verification in step e).

The stress model does not necessarily need to involve a neural network. It can merely be designed as an editable look-up table whose acoustic property threshold value, motion property threshold value and physiologic property threshold value are edited initially or can be replaced during a learning mode of the method.

In brief, the learning tracks what acoustic stimuli worked in what stress situation, and what did not work in what stress situation. Moreover, the stress model learns what acoustic stimulus was applied in what acoustic and emotional situation and whether it led to
i. a decreased re-measured acoustic property value and/or motion property value and/or physiologic property value compared to the acoustic property value, the motion property value and the physiologic property value measured at step a) and b), respectively, or
ii. the same acoustic property value and/or motion property value and/or physiologic property value compared to the acoustic property value, the motion property value and the physiologic property value measured at step a) and b), respectively, or
iii. an increased acoustic property value and/or motion property value and/or physiologic property value compared to the acoustic property value, the motion property value and the physiologic property value measured at step a) and b), respectively.

If it is desired to save electrical energy, it may be required to adjust the method such that the method re-starts at step a) again after step e) after a predefined time delay only. For example, that the method does not start before 3 minutes after the acoustic and/or emotional situation changed or remained unchanged.

Next let us revert to an embodiment of the method that is operable in an interactive learning mode. Such an embodiment is particularly useful, if no physiologic property value is available and such that the uncertainty on whether the stress situation was identified correctly is pertinent. In such a case, a response on whether the end user perceives any stress or not is obtained before step c) of the above-mentioned method is executed.

In case that the end user indicates that she/he does not perceive any stress, a stress model is updated with the following information before the method restarts at step a):
i. The acoustic property value measured at step a);
ii. The motion property value measured at step b);
iii. An acoustic property threshold value that is increased by a predefined increment compared to the acoustic property threshold value that was stored in the stress model for the same acoustic situation.

The above situation may be a so-called false positive stress detection. The interactive learning method is an elegant way of indicating such situations to the stress model to avoid any mistakes in an identical or comparable sound scene/emotional stress situation where the method is operated on the automatic mode or the self-learning mode.

In case that the end user indicates that she/he perceives any stress, a response on whether the end user perceives mainly acoustically induced stress or not is obtained by the stress model, too.

In case that the end user indicates that she/he perceives mainly acoustically induced stress, the stress model is updated with the information that the acoustic property value measured at step a) leads to acoustically induced stress, followed by step c) where the acoustic stimulus comprises the first mask, and wherein in case that the end user indicates that she/he does not perceives mainly acoustically induced stress, the stress model is updated with the information that the acoustic property value measured at step a) does not lead to acoustically induced stress, followed by step c) where the acoustic stimulus comprises the second mask.

Asking on whether the user perceives predominantly acoustically induced stress is considered as a question that is easier to answer than the alternative question on whether she/he perceives predominantly emotional stress.

The prompting of the end user with the questions and the obtaining of the user feedback is best done via a user interface. Since most modern hearing devices have only limited opportunities to feeding in end user information, it is preferred to correspond via a user interface on an external device. Since most end users have a smart phone nowadays, using an app of the smart phone as well as its keyboard as the communication interface is the preferred solution. However, alternative interfaces on a tablet or a personal computer of the end user or a relative or friend are conceivable and are still covered by the present invention.

Although an input of the user feedback in spoken form would be possible via the primary microphones of the hearing devices, such an option is less favored owing to the variety of languages, dialects, mumbled or unclear voice commands and many more.

Again, the stress model does not need to involve a neural network. It can be a mere editable look-up table whose acoustic property threshold value, motion property threshold value and physiologic property threshold value can be edited initially or replaced during the interactive learning mode.

To avoid that the method cycle gets stuck if the end user does not provide the answer to the questions within a predefined time window, the interactive learning mode is aborted and jumps to step a) of the above-mentioned method after the acoustic stimulus with the first mask is applied at step d). In an exemplary method operated in the interactive learning mode, that predefined time window is 3 minutes. In other embodiments, the predefined time window can be longer or shorter.

Since it is particularly difficult to verify on whether the applied acoustic stimulus was effective in mitigating the stress perception in cases where no physiologic input is available. In such cases, it is recommendable that the method comprises an additional step of obtaining user input from the end user on whether she/he perceives any stress relief after step e) of the above-mentioned method, or not. That way, the training and updating of the stress model with correct data is achievable.

In case that the end user indicates that she/he perceives any stress relief, the stress model is updated with the following information before the method restarts at step a):
i. The acoustic property value measured at step a);
ii. The motion property value measured at step b);
iii. The acoustic stimulus that worked for that acoustic situation,

In case that the end user indicates that she/he does not perceive any stress relief, the stress model is updated with the following information before the method continues at step c) with the same stimulus that led to the present situation, but with an increase of the intensity when applying the acoustic stimulus at step d):
i. The acoustic property value measured at step a);
ii. The motion property value measured at step b);
iii. The acoustic stimulus that did not work for that acoustic situation.

There may also be a so-called false negative situation where the end user indicates in the user feedback that she/he perceives a stress relief, but where the re-measured physiologic property value is equal or higher than the physiologic property value before applying the acoustic stimulus in step e). That is indicative that the acoustic stimulus selected at step c) did not work because the body response is regarded as a more reliable feedback source than any user feedback, in particular if physiologic property values are available. Hence, the interpretation is such that the stimulus did not work or that its application time was too short to show a physical effect by the end user, despite the user perception is different. In such an exceptional case, the user indication is ignored, and the same acoustic stimulus is re-applied in step e) of a subsequent method cycle for the same situation again, preferably with an increased intensity. The stress model is not updated. The cycle of the method is continued until the learning is aborted.

There may also be a situation where the end user indicates in the user input that she/he perceives stress, in particular acoustically induced stress, but where the measured physiologic property value is lower than the physiologic property threshold value before applying the acoustic stimulus in step d). In such a case, the acoustic property threshold value is decreased by a predefined increment compared to the acoustic property threshold value that was stored in the stress model for the same acoustic situation in the stress model because the end user's stress tolerance to acoustically induced stress proves to be lower than anticipated during the fitting process or previous training phases for the same sound scene as presently represented in the stress model. In an exemplary embodiment, the predefined increment is 1 dB.

In an alternative or a complementary embodiment, an altered or alternative acoustic stimulus can be applied in step d) next time when the interactive learning mode is active and in a comparable stress situation to test whether the end user just disliked the acoustic stimulus received even though it worked.

To prevent the stress model from being fed with defective or contradictory data, it is possible to activate additional rules such as the following, exemplary rule: Once the end user feedback indicates three times in a row for the same acoustic sound scene/emotional situation that she/he still perceives stress relief despite the body response shows a decreased stress reaction after applying the acoustic stimulus, and in a further option also the alterative acoustic stimulus or the alternative stimuli worked, then the learning process is aborted at least for a predefined amount of time and without any modification of the entries in the stress model. In an exemplary embodiment, the predefined amount of time is 5 minutes.

### BRIEF DESCRIPTION OF THE DRAWINGS

Reference will now be made in detail to embodiments, examples of which are illustrated in the accompanying drawings. The drawings illustrate various embodiments and are a part of the specification. The illustrated embodiments are merely examples and do not limit the scope of the disclosure. Throughout the drawings, identical or similar reference numbers designate identical or similar elements. In the drawings:
- Fig. 1: schematically illustrates a sectional view of an ITE-type hearing device in the ear canal of an end user;
- Fig. 2: illustrates a first portion of the flowchart of the present method;
- Fig. 3: illustrates a second portion of the flowchart of the present method;
- Fig. 4: illustrates a third portion of the flowchart of the present method; and
- Fig. 5: illustrates a fourth portion of the flowchart of the present method.

### DETAILED DESCRIPTION OF THE DRAWINGS

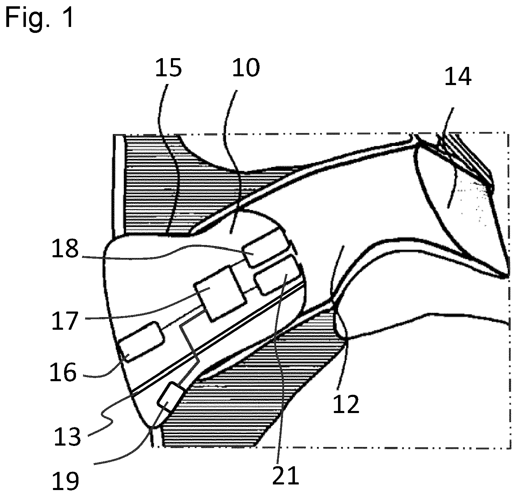

The hearing device 10 shown in **figure 1** is an ITE that is placed into an ear canal 12 of an end user. The hearing device 10 has a vent 13 that forms a channel extending between the inside of the ear canal and the outside and the ambient environment (i.e. the region of the pinna) and allows, amongst other effects, for a pressure equalization between those regions when the hearing device is worn. Vents are well-known means to lower undesired acoustic effects like occlusion and sound artifacts and contribute to a better-balanced microclimate with respect to humidity. Although an acoustically closed coupling is preferable in some use situations, an acoustically open coupling can be desired in other use situations (own-voice, environmental awareness). A vent renders a closed coupling towards an open coupling, dependent on the vent size.

The hearing device 10 is placed tightly in the ear canal 12 such that any gap 15 is avoided and that the hearing device 10 is sealed in the ear canal 12. The hearing device 10 has at least one primary microphone 16 that is configured to receive incoming sound and to concert it to a digital audio input. The audio input is then led to a sound processing unit 17. In the present embodiment, the sound processing unit 17 is also an intelligence unit that is configured to capture an acoustic property value 22 and comprises all electronics that is required to execute the method explained in further detail below, for example a look-up table, memory and many more as well as all electronics that is required to address the hearing impairment of the end user. Once the sound processing is concluded, the sound processing unit 17 produces a sound output signal that is led to a receiver 18 that converts it into sound waves again that are then led to the ear drum 14. As a result, the ear drum is receiving processed sound via the sound processing path with the sound processing unit 17 as well as direct sound via the direct sound path with the vent 13.

In the present embodiment of the hearing device 10, there is a heart rate sensor 19 provided and connected with the intelligence unit of the sound processing unit 17. The heart rate sensor 19 is required to capture a physiologic property value 20 in the form of a heart rate since the heart rate variability is forms a body reaction if the end user perceives stress. The hearing device 10 also has an accelerometer 21 that is configured to sensing a motion property value 23 that is indicative of head motions of the end user. The positions of the sensors in figure 1 are purely schematic and are place at optimal positions. In more advanced embodiments of the method, there may be more sensors to measure a plurality of physiologic property values. Such an embodiment offered the opportunity to enhance the accuracy of the stress detection despite a higher electrical energy consumption.

The hearing device is powered by a replaceable or a rechargeable battery that provides the electrical power for all processes running in the hearing device in an operating state of the hearing device (not shown in fig. 1).

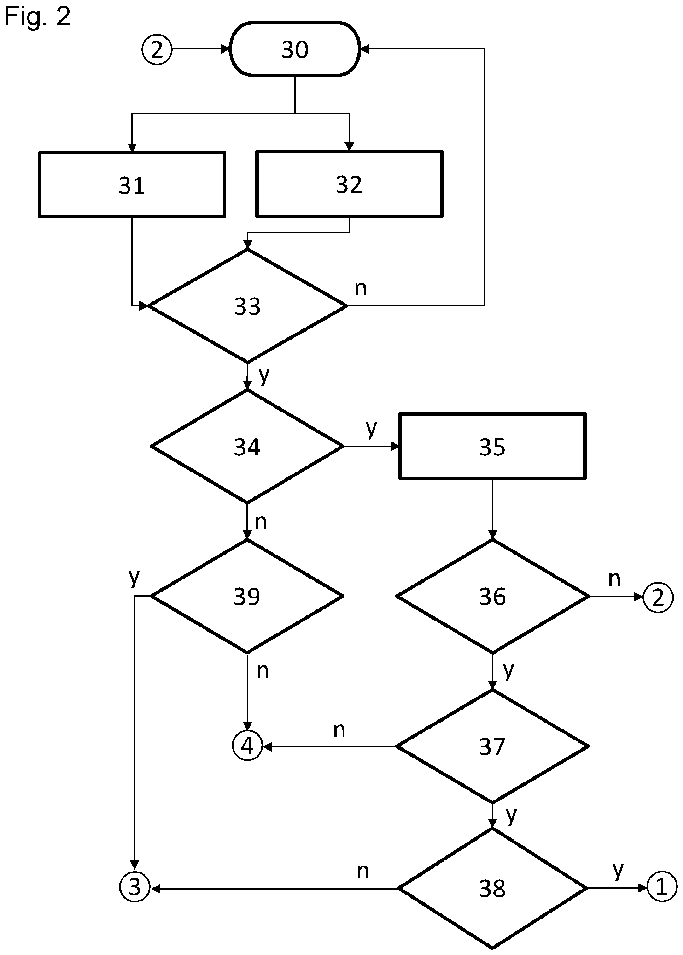

Now let us revert to the method for detecting stress and how to mitigate the effects for an end user by way of a flowchart shown in **figure 2** to **figure 5****.**

The method starts at step 30 in the flowchart on figure 2. Next, an acoustic property value 22 is measured by the processing unit 17 at step 31. Parallel thereto a motion property value 23 is measured by the accelerometer 21 at step 32. Next, the intelligence unit of the processing unit 17 compares the acoustic property value 22 with an acoustic property threshold value that is predefined or pre-learned earlier on and stored in the look-up table of the stress model. The acoustic property value measures the noise level (loudness), the sound pattern, the speech intensity as well as the sound pitch.

At step 33, the intelligence unit verifies whether the acoustic property value is equal to or higher than the acoustic property threshold value. If so, then the method continues by step 34. Parallel thereto, the intelligence unit of the processing unit 17 also compares the motion property value 23 with a motion property threshold value that is predefined or pre-learned earlier on and stored in the look-up table of the stress model, too. The motion property value is captured by an accelerometer that measures the head movements of the end user, i.e., their pattern, frequency and intensity. If the motion property value is equal to or higher than the motion property threshold value, then the method continues by step 34. To avoid any misunderstandings, the method continues with step 34 even if there is one of these two values is equal or higher than the predefined threshold values, each.

Since there is a heart rate sensor that is configured to measure a physiologic property value is provided, we enter the path of the flowchart to the next step 35 comprising the measurement of the physiologic property value. The path of the flowchart where there is no physiologic input sensor provided is explained later. Let us continue with the part of the flowchart where a physiologic input sensor is available.

At step 36, the intelligence unit verifies whether the physiologic property value is equal to or higher than a physiologic property threshold value that is predefined or pre-learned earlier on and stored in the look-up table of the stress model, too. Measuring the physiologic property value in addition to the acoustic property value and the acoustic property value not only increases the accuracy of the stress detection and the identification but also forms a prerequisite for a self-learning mode of the method where the stress model learns on whether the stimulus or stimuli applied led to the desired effect or whether the threshold values can be adapted, adjusted and replace the present threshold values stored in the look-up table of the stress model.

If the verification at step 36 reveals that the physiologic property value is lower than the physiologic property threshold value stored in the stress model, then the process starts anew at step 30. To save some electrical power, there is a time delay provided before the process re-starts at step 30 again.

After step 36, there is a first logic step 37 that verifies whether the method is operated any learning mode or not. There are two learning modes, the self-learning mode mentioned earlier on and an interactive learning mode that will be explained below in more detail. If no learning mode is activated, then the method must be running in the automatic mode and the next step in the flowchart is step 43.

In case that the method is not operated in any learning mode, the first logic step 37 detects that the method is operated in the automatic mode where the stress is mitigated by applying a stimulus or a mix of several stimuli depending on the detection of the kind/type of stress. In case the method is operated in any learning mode, the second logic step 38 detects if the method is operated in the self-learning mode or not. In case it is operating in the self-learning mode, the process continues at step 43. If the second logic step 38 detects that the method is not operated in the self-learning mode, then it must be operating in the interactive learning mode where the end user is involved to train the stress model and the next step in the flowchart is step 40.

Before leaving figure 2, let us revert to step 34. If the logic step detects that no physiologic sensor is available or if that sensor is defect and does not provide any physiologic property value any longer, then the process assumes that the end user perceives stress and proceeds with step 39 that verifies with a third logic step whether the method is operated in the interactive learning mode or not. If not, then the next step in the flowchart is step 43. If it is operated in the interactive learning mode, then then the next step in the flowchart is step 40.

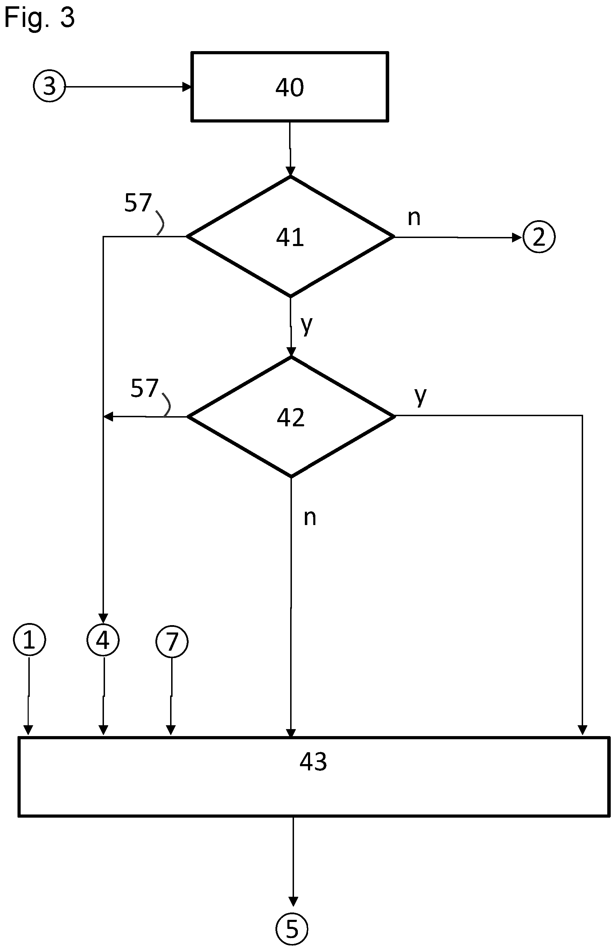

**Figure 3** addresses the interactive learning mode where the end user is involved for tailoring and/or adjusting the settings and values of the stress model via a user interface on her/his smartphone that is in communication with her/his hearing devices. At step 40, the end user is asked on whether she/he perceives stress. At discriminator step 41, there are three options available. The end user tells the stress model that she/he does not perceive stress, or she/he perceives stress. If there is no end user response received within a predefined amount of time, here in this exemplary embodiment 3 minutes, then the method swaps into the automatic mode via path 57. The end user may go back to the interactive learning mode via the user interface any time later.

In case that the end user indicates that she/he perceives stress, the stress model is updated accordingly. Depending on whether the acoustical property value and/or the motion property were equal to or above their threshold values, that threshold or thresholds are increased by a predefined increment of 1 dB loudness in this example and for this situation since the stress threshold for the user is obviously higher than anticipated or than it was in past cases. The model is updated with the following information:
- The acoustic property value measured at step a);
- The motion property value measured at step b);
- The physiologic property value measured at step b);
- In case that only the acoustic property value was higher than the acoustic property threshold value, the acoustic property threshold value is raised by 1 dB.
- In case that only the motion property value was higher than the acoustic property threshold value, the motion property threshold value is raised by 10 percent.
- In case that both the acoustic property value as well as the motion property value were equal to or above their dedicated threshold, then both the acoustic property threshold value and the motion property threshold value are raised by 1 dB and 10 percent, respectively.

Next, the workflow starts at step 30 again with an exemplary delay of 4 minutes to save electricity.

If the end user feedback tells the stress model that she/he perceives stress, then the end user is asked in step 42 on whether she/he perceives predominantly acoustically induced stress, i.e., environmental stress caused by sounds, not by emotions. This question is asked because it is easier to answer than whether is predominantly emotional stress that she/he perceives.

In case that the end user indicates that she/he perceives predominantly acoustically induced stress, the stress model is updated accordingly. Obviously, the stress threshold for the user is lower than anticipated or than it was in past cases. The model is updated with the following information:
- The acoustic property value measured at step a);
- The motion property value measured at step b);
- The physiologic property value measured at step b);
- A new acoustic property threshold value that is lowered by 1 dB compared to the acoustic property threshold value that was previously in the stress model for that acoustic sound scene/situation.

In case that the end user answers the question on whether the stress load was mainly caused by acoustically induced stress to the negative, the stress model is updated accordingly. Obviously, it must have been emotional stress that caused the stress reaction of the end user. The model is updated with the following information:
- The acoustic property value measured at step a);
- The motion property value measured at step b);
- The physiologic property value measured at step b);
- A new motion property threshold value that is lowered by 10 percent compared to the motion property threshold value that was previously in the stress model for that situation.

If there is no end user response received at step 42 within a predefined amount of time set to exemplary 3 minutes, then the method swaps via path 57 into the automatic mode and it is assumed that the stress was predominantly acoustically induced.

The end user may go back to the interactive learning mode via the user interface any time later.

Next, step 43 with the selection of the stimulus to be applied to mitigate the stress perceived by the end user is explained in more detail. There are three possible cases to be distinguished from one another at this step:

Scenario 1 resides in that at step 31, an acoustic property value was measured that proved at the verification step 33 to be equal to or higher than the acoustic property threshold value, whereas a motion property value was below the motion property threshold value. In this case, it is the acoustically induced stress that dominates. Hence, a first sound mask comprising exemplary wind noise is selected to form the acoustic stimulus to be applied to the processed sound. Scenario 1 also applies when the end user selected an interactive learning mode before, indicated that she/he experiences stress at step 41 but did not respond in time at step 42 on whether the stress perceived was mainly caused by acoustically induced stress or not. The too late or no response path is denoted by reference character 57.

Scenario 2 resides in that at step 31, a motion property value was measured that proved at the verification step 33 to be equal to or higher than the motion property threshold value, whereas an acoustic property value was below the acoustic property threshold value. In this case, it is the emotional stress that dominates. Hence, a second sound mask comprising exemplary wind noise is selected to form the stimulus to be applied to the processed sound.

Scenario 3 resides in that at step 31, an acoustic property value was measured that proved at the verification step 33 to be equal to or higher than the acoustic property threshold value, whereas the motion property value measured also proved to be equal to or higher than the motion property threshold value, In this case, the stimulus comprises both the first mask as well as the second mask. If there was a qualitative measurement of the acoustically induced stress share to the emotional stress share it was possible to apply a mix with different weights for the first mask and the second mask. For the sake of simplicity, let us assume a basic embodiment of the method where the ratio of the first mask to the second mask is one to one.

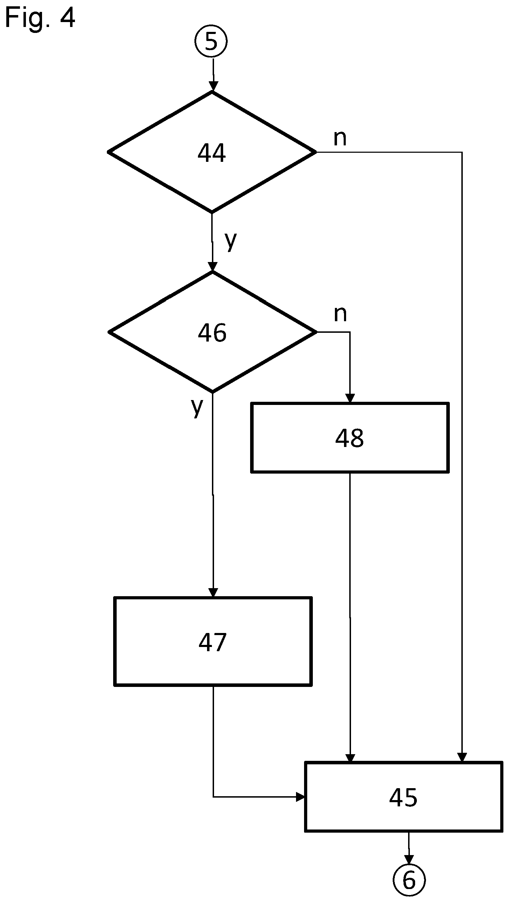

**Figure 4** addresses the closer evaluation and application strength of the stimulus to be applied at step d) of the method for modifying the sound processing of the audio input. At step 44, the method checks whether the same stimulus was applied already at a predeterminable time before, here an exemplary 1 minute ago.

If that was not the case and the stimulus selected at step c) of the method was not applied within the last minute, then it is applied at step 45. The application is a weighted mixing of the processed sound signal in the processing path and the stimulus selected at step c) of the method. The mixing and the modification of the audio input takes place at the sound processing unit 17.

If the stimulus selected at step c) of the method was selected to mitigate the stress of the end user in the same or almost the same stress situation, regardless whether acoustically induced and/or emotional stress, then the flowchart path leads to the second discriminator step 46 that verifies whether the very same stimulus was already applied several times, let us assume 20 times during the past 5 minutes for the same stress situation or not.

In case that step 46 reveals that the same stimulus was already applied several times but obviously did not lead to a decrease of the motion property value or the physiological property value, then it is assumed that said stimulus is not optimal and he is replaced by an alternative stimulus at a stimulus replacement step 47 before the alternative stimulus is applied at step d) of the method.

In case that the method is operated in the self-learning mode or the interactive learning mode, the stress model is updated with the following information:
- The acoustic property value measured at step a);
- The motion property value measured at step b);
- The physiologic property value measured at step b);
- The lesson learned that the stimulus did not lead to a decrease of the stress;
- The alternative stimulus that is selected and applied, now.

In case that step 46 reveals that the same stimulus was already applied before but has not reached the predefined repetition threshold, yet (here exemplary 20 times during the past 5 minutes) the same stress situation obviously continues to exist. Let us assume in this example that it is the fourth time that the same stimulus was applied before, i.e., in preceding cycles and for the same stress situation. Now, the intensity of the selected stimulus is intensified. In a basic example, the loudness is increased by 1 dB at an intensity increase step 48. In an alternative embodiment, the sound sharpness is increased by 10 percent. In yet another embodiment, an active noise cancelling is activated. It is possible to modify the stimulus by combining several measures.

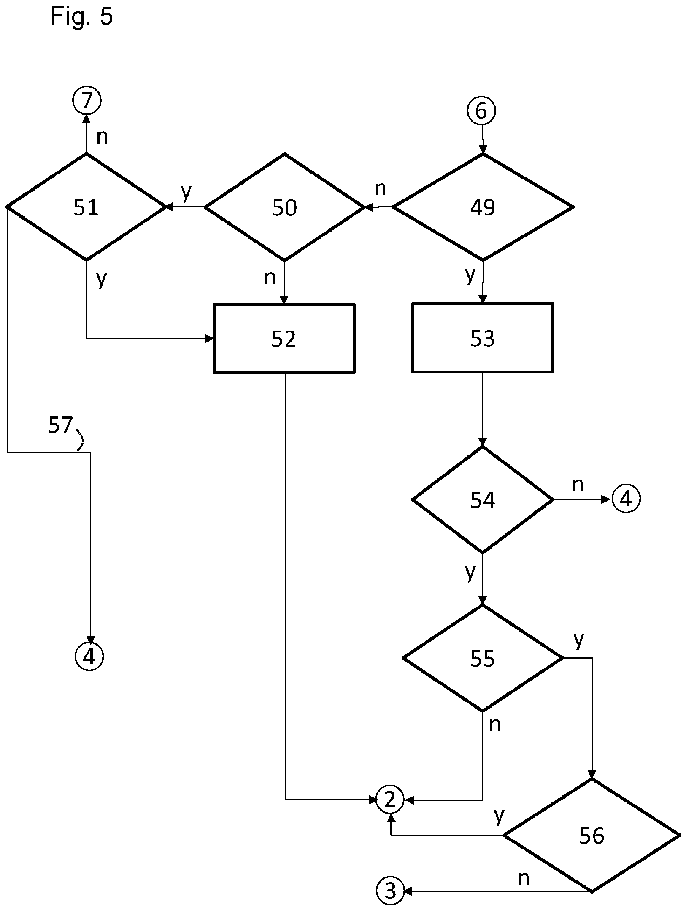

**Figure 5** shows the situation after the application of the stimulus at step 45 and involves the verification on whether the stimulus led to the desired mitigation of the stress experienced by the end user and drawing the conclusions in case of operating the method in the self-learning mode. The flowchart is somewhat complex because it must cover all operating modes of the method, i.e., the automatic mode, the self-learning mode as well as the interactive learning mode. Because the latter can imply the absence of a physiologic input sensor as well as a deliberate operating choice of the end user while there is a physiologic input sensor, the method needs to cover all cases.

After the stimulus was applied, it is first verified at step 49 whether a physiologic property value was available or not. At this step 49, the information from step 34 can be re-used.

In case that step 49 proves that there is no physiologic property value was available, a third logic step 50 verifies whether the interactive learning mode is activated or not. In case it is activated, there the end user is actively asked by the stress model at step 51 either acoustically and/or via the user interface on whether the user perceived any stress relief after the application of the stimulus, or not.

If the end user indicates at step 51 that she/he did not perceive any stress reduction, then the process proceeds by the selection of the stimulus at step 43 again.

If there is no end user response received at step 51 within a predefined amount of time set to 3 minutes in this embodiment, then the method swaps via path 57 into the automatic mode and it is assumed that the stress persists. Hence, the process proceeds by the selection of the stimulus at step 43 again.

In case that the third logic step 50 detects that no interactive learning is activated, the method must be in the automatic mode. This because the self-learning mode is not possible in absence of a physiologic property value. In this case, it is assumed that the stress persists, and the same stimulus is re-applied for a predefined number of times and/or for a predefined amount of time. In this example, the stimulus is re-applied 20 times in a time window of 5 minutes at an application step 52 before the process re-starts at step 30 again. As before, there can be a predefined delay in time before the process re-starts at step 30 again.

The same re-application of the repetition at step 52 applies if the end user indicated at step 51 to the stress model that she/he does not perceive any stress relief after the application of the stimulus. This is possible because it may take some time to detect a change of the body signals and thus the acoustic property value and/or the motion property value and/or the physiological property value to react to the stimulus. Different to step 50, the stress model is updated with the following information in case that the interactive learning mode is activated, and the user indicates that there is no stress relief perceived:
- The acoustic property value measured at step a);
- The motion property value measured at step b);
- The physiologic property value measured at step b);
- The acoustic property value re-measured at step e);
- The motion property value re-measured at step e);
- The stimulus that was applied;
- The lesson learned that the stimulus did not lead to a decrease of the stress after the given amount/number of applications or re-applications.

In this case, the stimulus is re-applied 20 times in a time window of 5 minutes at an application step 52 before the process re-starts at step 30 again. As before, there can be a predefined delay in time before the process re-starts at step 30 again.

Now let us focus on the case where at step 49 there is a physiologic property value available. In this case, the re-measurement step 53 where the physiologic property value is re-measured according to step e) of the method is performed. Once that re-measurement took place, a third discriminator step 54 verifies if the re-measured physiologic property value at step e) is lower than the physiologic property value measured at step b) before.

If the third discriminator step 54 detects that the re-measured physiologic property value at step e) is equal or even higher than the physiologic property value measured at step b) before, then the stimulus has either not been applied sufficiently long enough or it was unsuitable to lower the stress level of the end user. In both cases, the flowchart proceeds by step 43.

If the third discriminator step 54 detects that the re-measured physiologic property value at step e) is lower than the physiologic property value measured at step b) before, then there is a differentiation needed that addresses the presence of any activated learning modes of the method because in such case there will be an update of the stress model.

Step 55 verifies on whether the method is presently run in any learning mode, regardless of whether in the self-learning or the interactive learning mode, or not. In case that the method is not operated in any learning method, it must be activated in the automatic mode. Hence, the flowchart proceeds by the re-starting of the process at step 30. As before, there can be a predefined delay in time before the process re-starts at step 30 again.

If the verification at step 55 proves that a learning mode is activated, one needs to know which learning mode as the procedure is different. This question on whether the method is presently operated in the self-learning mode or not is performed at step 56.

In case that step 56 reveals that the self-learning mode is activated, the stress model is updated with the following information:
- The acoustic property value measured at step a);
- The motion property value measured at step b);
- The physiologic property value measured at step b);
- The acoustic property value re-measured at step e);
- The motion property value re-measured at step e);
- The physiologic property value re-measured at step e);
- The stimulus that was applied;
- The lesson learned that the stimulus did lead to a decrease of the stress after the given amount/number of applications or re-applications.

In case that step 56 reveals that the method is not operated in the self-learning mode, it must be operating in the interactive learning mode. Hence, the process of the flowchart leads to steps 40, 41 and 42 again, where the end user is asked on whether she/he still perceives stress, and if so, whether it is believed to be mainly acoustically induced stress or not. If the end user indicates that she/he perceives stress reduction/stress relief, but where the re-measured physiologic property value is equal or higher than the physiologic property value before applying the acoustic stimulus in step e) of the method, then the acoustic stimulus is re-applied in step e) in a subsequent method cycle for the same situation again. In such a case, the stress model is not updated because the end user's perception and the re-measured values would be contradictory with respect to their meaning. In this case, the process proceeds with step 43.

If the end user indicates that she/he perceives no stress reduction/stress relief, but where the re-measured physiologic property value is actually lower than the physiologic property value before applying the acoustic stimulus in step e) of the method, then the acoustic stimulus is believed to work as desired and is thus re-applied in step e) in a subsequent method cycle for the same situation again for a predefined number of times or an amount of time such as defined in step 52 such that the stress is mitigated safely below the predefined threshold value. The trustfulness of the body reaction is in this case regarded as the more accurate information than the end user feedback. In such a case, the stress model is not updated because the end user's perception and the re-measured values would be contradictory with respect to their meaning. In this case, the process proceeds with step 43.

If the end user indicates that she/he perceives a stress reduction/stress relief, and where the re-measured physiologic property value is actually lower than the physiologic property value before applying the acoustic stimulus in step e) of the method, then the acoustic stimulus works and is thus re-applied in step e) in a subsequent method cycle for the same situation again for a predefined number of times or an amount of time such as defined in step 52 such that the stress is mitigated safely below the predefined threshold value. In such a case, the stress model is updated with the following information:
- The acoustic property value measured at step a);
- The motion property value measured at step b);
- The physiologic property value measured at step b);
- The acoustic property value re-measured at step e);
- The motion property value re-measured at step e);
- The physiologic property value re-measured at step e);
- The stimulus that was applied;
- The lesson learned that the stimulus did lead to a decrease of the stress after the given amount/number of applications or re-applications.

If there is no end user response received at step 41 or 42 within a predefined amount of time set to 3 minutes in this embodiment, then the method swaps via path 57 into the automatic mode and it is assumed that the stress persists. In this case, the process proceeds by the selection of the stimulus at step 43 again.

### EXAMPLE 1:

The sequence of events is as follows:
- The end user is male, 50 years old, healthy but with a light hearing impairment.
- The potential stressful situation is caused by the sound of a circular/buzz saw shrieking with an irregular pattern and sound pitches at about 60 dB and in a range of 4-5 kHz for 5 minutes.
- A physiologic sensor in the form of a heart rate sensor is built into both hearing devices of the end user.
- The method according to the present invention is operating in the automatic mode.
- After 2 minutes, the heart rate value of the end user is above the predefined threshold of 100 heart beats at a 122 heart beats. The algorithm that performs the method according to this invention is started.
- The exposure to the buzz saw noise together with the elevated heart rate is interpreted as acoustically induced stress. At step c) of the method, the stimulus selected comprises a first mask that is configured to reduce noise in the audio input since that is a good method to mitigate acoustically induced stress.
- At step d), the stimulus is added to the processed sound signal with a weighting of 50%.
- At step e), the method re-measures the heartbeat rate of the end user. It is unchanged. The same stimulus designed for the noise reduction is applied with 75% weighting for another two minutes, i.e., with an increased intensity.
- After two minutes, the heart rate is at 100.
- The buzz saw sound stops.
- The same stimulus is continuously applied with a time gap of 5 seconds between subsequent cycles of the method for two minutes.
- The heart rate is now at 95 and the method stops applying the stimulus because the heart rate is below the predefined threshold for a while, now.

### EXAMPLE 2:

The sequence of events is as follows:
- The end user is female, 60 years old, has a with a moderate hearing impairment and a light anxiety disorder.
- The potential stressful situation is caused in that the woman got her mail and there were letters from the tax office and many other bills. Because her pension is low, she starts breathing heavily.
- A motion sensor is built into both hearing devices of the end user.
- A physiologic sensor in the form of a heart rate sensor is built into the hearing devices of the end user.
- The method according to the present invention is operating in the automatic mode.
- The motion sensor detects the repetitive up and down of the head that is accompanying the end user's accelerated breathing and starts the algorithm that performs the method according to this invention.
- The audio input has a loudness that is below the predetermined acoustic property threshold value of 70 dB.
- After 2 minutes, the end user's heart rate value is above the predefined threshold of 100 heart beats at a 112 heart beats.
- The head movements and the absence of excessive acoustically induced stress along with the heart rate is interpreted as emotional stress.
- At step c), the stimulus selected is a second mask that is configured to mitigate emotional stress. The second mask comprises irregular wind sound rustling through tree leaves and is 1 dB loud.
- At step d), the stimulus is added to the processed sound signal with a weighting of 5% only.
- At step e), the method re-measures the heart rate of the end user. It is unchanged. The same stimulus is then applied again with a time gap of 5 seconds between subsequent cycles of the method for two minutes. The intensity of the stimulus is not altered.
- The end user calms down and after two minutes, the heart rate is at 102.
- The same stimulus is continuously applied with a time gap of 5 seconds between subsequent cycles of the method for two minutes.
- The heart rate is now at 96 and the method stops applying the stimulus because the heart rate is below the predefined threshold for a while, now.

### LIST OF REFERENCE SYMBOLS

- 10: hearing device
- 12: ear canal
- 13: vent
- 14: ear drum
- 15: gap
- 16: primary microphone
- 17: sound processing unit
- 18: receiver
- 19: heart rate sensor
- 20: physiological property value
- 21: accelerometer
- 22: acoustic property value
- 23: motion property value
- 30: start
- 31: measuring an acoustic property value
- 32: measuring a motion property value
- 33: verifying whether at least one of the acoustic property value and the motion property value is equal to or above its dedicated acoustic property threshold value and motion property threshold value, respectively
- 34: verification if there is a physiologic sensor / physiologic property value available
- 35: measuring a physiologic property value
- 36: verifying whether the physiologic property value is equal to or above the physiologic property threshold value
- 37: first logic step - check whether any learning mode is activated
- 38: second logic step - check if the self-learning mode is activated
- 39: third logic step - check if the self-learning mode is activated
- 40: asking the end user whether she/he perceives stress
- 41: discriminator step with the user response to the stress question
- 42: asking the end user whether she/he perceives predominantly acoustically induced stress or not
- 43: selecting a stimulus
- 44: verification on whether the same stimulus was applied before
- 45: application of the stimulus to the processed sound signal
- 46: second discriminator step
- 47: stimulus replacement step
- 48: intensity increase step
- 49: verification whether a measured and a re-measured physiologic property value is/was available
- 50: third logic step - check if the interactive learning mode is activated
- 51: asking the end user whether she/he perceived any stress relief
- 52: application step
- 53: re-measurement of the physiological property value
- 54: third discriminator step - check if the re-measured physiological property value is lower than the physiological property value measured at step 35
- 55: fourth discriminator step - check whether any learning mode is activated
- 56: verification step - check whether the self-learning mode is activated
- 57: too late or no response path

## Claims

1. A method for detecting a sensory overload of a user with a hearing device (10) by means of a detection system, the method comprising the steps of
a. Measuring (31) an acoustic property value (22) of an audio input via the hearing device (10), which acoustic property value (22) is serving as an identifier of the presence of an acoustically induced stress,
b. Measuring (32) a motion property value (23) serving as an identifier of the presence of a behavioral stress of the user,
c. Selecting (43) an acoustic stimulus that is configured to modify a sound processing of the audio input and convert it to an audio output if the acoustic property value is equal to or above a predetermined acoustic property threshold value and/or if the motion property value is equal to or above a predetermined motion property threshold value,
d. Modifying the sound processing of the audio input by applying the acoustic stimulus (45),
e. Re-measuring the acoustic property value and the motion property value and verifying whether the re-measured motion property value is lower than the motion property value measured at step b).

2. Method according to claim 1 or 2, **characterized in that** the acoustic stimulus comprises at least one of a first mask that is configured to mitigate acoustically induced stress, and a second mask that is configured to mitigate emotional stress.

3. Method according to claim 1 or 2, **characterized by** an additional step of measuring (35) a physiologic property value (20) in addition to the motion property value (22) in step b) of claim 1, and
by considering said physiologic property value (20) when selecting (43) the acoustic stimulus at step c) of claim 1, and
by re-measuring (53) the physiologic property value at step e) of claim 1 in addition to the re-measuring of the motion property value, and
by verifying (54) whether the re-measured physiologic property value is lower than the physiologic property value measured at step b) of claim 1.

4. Method according to any one of claim 1 to 3, **characterized in that** the acoustic stimulus is re-selected at step c) of claim 1 and re-applied at step d) of claim 1 in a new cycle of the method that is following a previous cycle of the method if the re-measured acoustic property value, re-measured motion property value, and if available the re-measured physiologic property value at step e) of claim 1 of the new cycle of the method is lower than the acoustic property value, the motion property value, and if available the physiologic property value measured at step a) and b) of claim 1 of the previous cycle of the method, respectively.

5. Method according to any one of claim 1 to 4, **characterized in that** the acoustic stimulus is re-selected at step c) of claim 1 and re-applied with an increased intensity at step d) of claim 1 in a new cycle of the method if the re-measured acoustic property value, re-measured motion property value, and if available the re-measured physiologic property value at step e) of claim 1 of the new cycle of the method is equal to or higher than the acoustic property value, the motion property value, and if available the physiologic property value measured at step a) and b) of claim 1 of a previous cycle of the method, respectively.

6. Method according to any one of claims 4 or 5, **characterized in that** the acoustic stimulus is replaced at step c) of claim 1 in a new cycle of the method by an alternative acoustic stimulus, if the re-measured acoustic property value, re-measured motion property value, and if available the re-measured physiologic property value at step e) of claim 1 of the new cycle of the method is equal to or higher than the acoustic property value, the motion property value, and if available the physiologic property value measured at step a) and b) of claim 1 of a previous cycle of the method, respectively, and
provided that several repetition cycles with the acoustic stimulus exceeded a predefined repetition threshold.

7. Method according to anyone of claims 4 to 6, **characterized by** an additional step of aborting the re-execution of the method if a predefined further repetition threshold is exceeded.

8. Method according to any one of claims 1 to 7, **characterized in that** when the method is operable in an automatic mode, where the steps of the method are repeated until an operating mode other than the automatic mode is selected, and/or **in that** the method re-starts at step a) of claim 1 in case the re-measured physiologic property value at step e) of claim 1 of an initial cycle of the method is lower than a predefined physiologic property threshold value.

9. Method according to any one of claims 1 to 7, **characterized in that** when the method is operable in self-learning mode, where a stress model is updated with the following information after executing step e) of claim 1:
a. The acoustic property value measured at step a) of claim 1;
b. The motion property value measured at step b) of claim 1;
c. The physiologic property value measured at step b) of claim 1;
d. what acoustic stimulus was applied in what intensity in step d) of claim 1;
e. The re-measured acoustic property value measured at step e) of claim 1;
f. The re-measured motion property value measured at step e) of claim 1;
g. The re-measured physiologic property value measured at step e) of claim 1;
h. A conclusion derived based on a result of the verification in step e) of claim 1.

10. Method according to any one of claims 1 to 10, **characterized in that** the method restarts at step a) of claim 1 again after step e) of claim 1 after a predefined time delay.

11. Method according to claim 2, **characterized in that** the method is operable in an interactive learning mode, wherein a response on whether the end user perceives any stress or not is obtained before step c) of claim 1 is carried out,
wherein in case that the end user indicates that she/he does not perceive any stress, a stress model is updated with the following information before the method restarts at step a) of claim 1:
a. The acoustic property value measured at step a) of claim 1;
b. The motion property value measured at step b) of claim 1;
c. An acoustic property threshold value that is increased by a predefined increment compared to the acoustic property threshold value that was stored in the stress model for the same acoustic situation,
and wherein in case that the end user indicates that she/he perceives any stress, a response on whether the end user perceives mainly acoustically induced stress or not is obtained,
wherein in case that the end user indicates that she/he perceives mainly acoustically induced stress, the stress model is updated with the information that the acoustic property value measured at step a) of claim 1 leads to acoustically induced stress, followed by step c) of claim 1 where the acoustic stimulus comprises the first mask, and
wherein in case that the end user indicates that she/he does not perceive mainly acoustically induced stress, the stress model is updated with the information that the acoustic property value measured at step a) of claim 1 does not lead to acoustically induced stress, followed by step c) of claim 1 where the acoustic stimulus comprises the second mask.

12. Method according to claim 11, **characterized in that** the method comprises an additional step of obtaining user input from the end user on whether she/he perceives any stress relief after step e) of claim 1 or not,
wherein in case that the end user indicates that she/he perceives any stress relief, the stress model is updated with the following information before the method restarts at step a) of claim 1:
a. The acoustic property value measured at step a) of claim 1;
b. The motion property value measured at step b) of claim 1;
c. The acoustic stimulus that worked for that acoustic situation,
and wherein in case that the end user indicates that she/he does not perceive any stress relief, the stress model is updated with the following information before the method continues at step c) of claim 1 with the same stimulus that led to the present situation, but with an increase of the intensity when applying the acoustic stimulus at step d) of claim 1:
d. The acoustic property value measured at step a) of claim 1;
e. The motion property value measured at step b) of claim 1;
f. The acoustic stimulus that did not work for that acoustic situation.

13. Method according to claim 12, **characterized in that** in a situation where the end user indicates in the user input that she/he perceives stress relief, but where the re-measured physiologic property value is equal or higher than the physiologic property value before applying the acoustic stimulus in step e) of claim 1, then the acoustic stimulus is re-applied in step e) of claim 1 in a subsequent method cycle for the same situation again.

14. Method according to claim 12, **characterized in that** in a case where the end user indicates in the user input that he/she perceives stress but where measured physiologic property value is lower than the predefined physiologic property threshold value existing at that time before applying the acoustic stimulus in step d) of claim 1, the acoustic property threshold value is decreased by a predefined increment compared to the acoustic property threshold value that was stored in the stress model for the same acoustic situation in the stress model.
